# FASCICULE DE BREVET EUROPEEN

(11) **EP 2 167 016 B1**
(45) Date de publication et mention de la délivrance du brevet: **27.10.2010**
(21) Numéro de dépôt: 08806138.7
(22) Date de dépôt: 01.07.2008
(51) Int. Cl.: A61K 8/02, A61K 8/73, A61K 9/06, A61K 9/19, A61K 31/722, A61Q 19/00, A61K 36/28, A61P 17/02

(54) **HYDROGEL DE CARBOXYALKYLAMIDE DE CHITOSAN, SA PREPARATION ET SON UTILISATION COSMETIQUE ET DERMATOLOGIQUE**
CHITOSAN-CARBOXYALKYLAMID-HYDROGEL, SEINE HERSTELLUNG UND SEINE KOSMETISCHE UND DERMATOLOGISCHE VERWENDUNG
CHITOSAN CARBOXYALKYLAMIDE HYDROGEL, PREPARATION THEREOF AND COSMETIC AND DERMATOLOGICAL USE THEREOF

(30) Priorité: 12.07.2007 FR 0705049
(43) Date de publication de la demande: 31.03.2010
(73) Titulaire: Biopharmex Holding Limited, Hong Kong (CN)
(72) Inventeur: LAUGIER, Elisabeth, CH-1207 Genève (CH); GOUCHET, Franck, F-41210 Marcilly En Gault (FR); PERRAUD, Jean-Pierre, F-75008 Paris (FR)
(74) Mandataire: Cabinet Plasseraud
(86) Numéro de dépôt international: PCT/FR2008/051214
(87) Numéro de publication internationale: WO 2009/007635

(56) Documents cités:
- WO-A-03/068281
- US-A1- 2005 113 773
- KIYOZUMI TETSURO ET AL: "The effect of chitosan hydrogel containing DMEM/F12 medium on full-thickness skin defects after deep dermal burn." BURNS : JOURNAL OF THE INTERNATIONAL SOCIETY FOR BURN INJURIES AUG 2007, vol. 33, no. 5, août 2007 (2007-08), pages 642-648, XP002467841 ISSN: 0305-4179
- BOUCARD NADÈGE ET AL: "The use of physical hydrogels of chitosan for skin regeneration following third-degree burns." BIOMATERIALS AUG 2007, vol. 28, no. 24, août 2007 (2007-08), pages 3478-3488, XP002467842 ISSN: 0142-9612
- SUH J K ET AL: "Application of chitosan-based polysaccharide biomaterials in cartilage tissue engineering: a review." BIOMATERIALS DEC 2000, vol. 21, no. 24, décembre 2000 (2000-12), pages 2589-2598, XP002467843 ISSN: 0142-9612

## Description

La présente invention concerne un hydrogel thixotrope de carboxyalkylamide de chitosan destiné au traitement cosmétique et dermatologique de brûlures cutanées.

La chitine est, avec la cellulose, un des polymères naturels les plus répandus. On l'obtient par exemple à partir de l'exosquelette de certains crustacées et insectes. La chitine est constituée de motifs de N-acétylglucosamine reliés entre eux par des liaisons bêta-1,4. Le chitosan est le produit de désacétylation par hydrolyse de la chitine, cette hydrolyse convertissant au moins une partie des motifs de N-acétylglucosamine en motifs glucosamine. On s'accorde généralement à désigner par le terme « chitosan » la chitine présentant un taux de désacétylation supérieur à 50 %. La désacétylation de la chitine augmente généralement la solubilité dans l'eau du polymère, mais cette solubilisation devient alors dépendante du pH, autrement dit le chitosan présentant un taux de désacétylation élevé, n'est soluble qu'en milieu acide, généralement à un pH inférieur à 6, lorsqu'une fraction suffisante de ses fonctions amine sont protonées.

L'excellente biocompatibilité de ces deux polymères et la biodégradabilité de la chitine et, dans une moindre mesure, du chitosan sont connues depuis longtemps. On a en outre décrit les propriétés bactériostatiques et fungistatiques de ces polymères. On a par ailleurs mis en évidence leur pouvoir d'activation de la cicatrisation de lésions cutanées chroniques ou aigües, superficielles (épidermiques) ou profondes, c'est-à-dire touchant au derme.

Ainsi, la demande de brevet français FR 2 736 835 divulgue un pansement pour plaies chroniques à base de chitine ayant un taux de désacétylation au plus égal à 40 %. Ce pansement se présente sous forme d'un hydrogel transparent relativement rigide.

La demande internationale WO 03/068281 divulgue un pansement, non pas à base de chitine, mais à base de chitosan ayant un taux de désacétylation au moins égal à 60 %, de préférence compris entre 94 et 98 %. Ce pansement se présente également sous la forme d'une plaque d'une épaisseur comprise entre 1 et 10 mm, éventuellement immobilisée sur un support, ayant une tenue mécanique suffisante pour pouvoir être manipulée et posée sur la plaie à protéger et à traiter.

Enfin, le brevet US 6 124 273 divulgue un hydrogel à base de chitine réticulée contenant un principe actif, en particulier une protéine, destiné à être libéré vers la plaie.

La présente invention avait pour but de mettre à profit les propriétés cicatrisantes connues du chitosan non pas sous la forme d'un pansement à base d'hydrogel relativement rigide tel que décrit dans les documents de l'état de la technique ci-dessus, mais sous forme d'un produit plus facile d'utilisation, susceptible d'être appliqué aisément, comme une crème ou une pommade, si nécessaire sur des zones relativement larges de la peau. Une telle utilisation permettrait une utilisation du chitosan non seulement en tant que pansement à usage médical, mais également pour le traitement cosmétique de brûlures légères superficielles, dues par exemple à une exposition prolongée au soleil.

La Demanderesse s'est donc fixé pour but de mettre au point un hydrogel de chitosan thixotrope, c'est-à-dire un gel physique (par opposition à un gel chimique avec des points de réticulation par liaison covalente) qui, au repos, a la consistance d'un gel, mais qui, lorsqu'il est soumis à des forces de cisaillement, présente une viscosité suffisamment faible pour pouvoir être étalé facilement sur de grandes étendues de la peau. Cet hydrogel devra avoir un pH relativement proche de celui de la peau, de préférence compris entre 6,5 et 7,2. En effet, si des valeurs de pH en dehors de cette zone sont tout à fait compatibles avec une application sur la peau saine, la Demanderesse a constaté que le soulagement rapide et efficace de la douleur causée par des brûlures, superficielles ou profondes, n'était obtenu qu'au prix d'un ajustement du pH du produit dans une gamme proche de celui de la peau.

La Demanderesse a alors rencontré le problème de la solubilité insuffisante du chitosan faiblement acétylé dans cette gamme de pH. En effet, lors de la neutralisation d'une solution acide de chitosan, un précipité se forme à pH d'environ 5 à 6 et la préparation d'un hydrogel thixotrope s'avère impossible.

La présente invention est basée sur la découverte qu'il était possible de préparer un hydrogel thixotrope, ayant la consistance d'une crème ou pommade, présentant un pH proche de celui de la peau (pH = 6,9) en convertissant au moins 40 %, de préférence au moins 50 % des fonctions amine des motifs glucosamine d'un chitosan ayant un taux de désacétylation d'au moins 85 %, en fonctions COOH en les faisant réagir avec un anhydride d'un diacide carboxylique approprié.

La présente invention a par conséquent pour objet un hydrogel thixotrope de carboxyalkylamide de chitosan, caractérisé par le fait qu'il présente une valeur de pH proche de celui de la peau, compris entre 6,5 et 7,2, de préférence entre 6,8 et 7, 0, et par le fait que ledit carboxyalkylamide de chitosan est constitué
- de 40 à 90 % en moles, de préférence de 50 à 80 % en moles, et en particulier de 50 à 75 % en moles, rapporté au nombre total de motifs (A) et (B), de motifs N-carboxyalkylamide de D-glucosamine de formule (I) (motifs (A)) 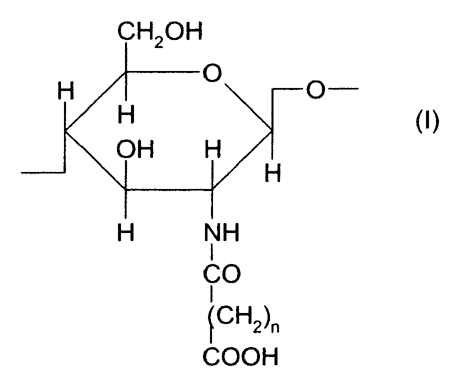 où n représente un nombre entier allant de 1 à 8, de préférence de 1 à 4, et en particulier égal à 2, ou un sel d'addition d'une base physiologiquement acceptable de celui-ci,
- de 60 à 10 % en moles, de préférence de 50 à 20 % en moles, en particulier de 50 à 25 % en moles, rapporté au nombre total de motifs (A) et (B), de motifs D-glucosamine protonés de formule (II) (motifs (B)) 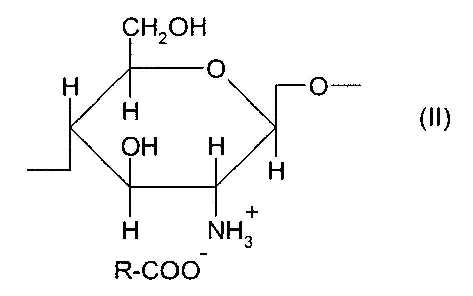 dans laquelle R représente un résidu alkyle en C₁₋₄, et
- de 5 à 15 % en moles, rapporté au nombre total de motifs (A), (B) et (C), de motifs N-acétyl-D-glucosamine (motifs (C)).

Comme indiqué ci-dessus un tel hydrogel doit être thixotrope, c'est-à-dire avoir une viscosité qui, en l'absence de forces de cisaillement, tend vers l'infini, c'est-à-dire le gel ne s'écoule pas ou très lentement. Lorsqu'il est soumis à des forces de cisaillement, par exemple lors de l'étalement sur la peau, sa viscosité diminue. La viscosité Brookfield du gel thixotrope de la présente invention, mesurée au moyen d'un viscosimètre Brookfield (aiguille n° 4, 30 - 60 tours/minute, 20 °C) est de préférence comprise entre 200 et 8000 centipoises, en particulier entre 300 et 1000 centipoises.

Le chitosan formant l'hydrogel de la présente invention est donc un sel d'addition d'acide d'un produit d'acétylation du chitosan par un anhydride d'un diacide carboxylique en C₃₋₁₀. L'acide utilisé pour acidifier les fonctions amine du chitosan n'ayant pas réagi avec l'anhydride est un acide organique de formule RCOOH où R représente un groupe alkyle en C₁₋₄. De préférence l'acide organique utilisé pour cette acidification est l'acide acétique, c'est-à-dire R représente un groupe méthyle. Cet acide est généralement utilisé lors de la dissolution du chitosan dans l'eau avant la réaction avec l'anhydride d'acide et la neutralisation du carboxyalkylamide de chitosan obtenu en tant que produit réactionnel.

Pour obtenir un hydrogel d'une consistance appropriée, la concentration du carboxyalkylamide de chitosan est de préférence comprise entre 0,5 et 3 % en poids, en particulier entre 1 et 2 % en poids. Une concentration inférieure à 0,5 % donnera généralement un gel trop peu visqueux qui s'écoule, même en l'absence de forces de cisaillement, alors qu'une concentration au-delà de 3 % en poids aboutit à des gels trop rigides du type de ceux décrits dans FR 2 736 835 et WO 03/068281, et qui ne se prêtent pas à un étalement aisé sur des zones importantes de la peau.

Les teneurs appropriées en carboxyalkylamide de chitosan indiquées ci-dessus dépendent bien entendu de la masse moléculaire moyenne des dérivés de chitosan utilisés. Plus la masse du polymère est importante, plus la concentration nécessaire pour obtenir une consistance appropriée est faible.

Les carboxyalkylamides de chitosan formant l'hydrogel de la présente invention ont généralement une masse moléculaire moyenne en poids comprise entre 10 000 et 800 000 Dalton, de préférence entre 50 000 et 200 000 Dalton.

Cette masse moléculaire peut être réduite, en cours de préparation de l'hydrogel, en traitant la suspension aqueuse ou la solution acide de chitosan avec de l'eau oxygénée.

L'hydrogel de la présente invention contient de préférence, en plus de l'eau, un agent de texture choisi parmi les polyols, destiné à assurer un bon étalement du gel sur la peau. L'hydrogel de la présente invention contient de préférence de 0,1 à 40 % en poids, en particulier de 0,1 à 20 % en poids, d'au moins un polyol. Le polyol préféré est le glycérol.

L'hydrogel de carboxylamide de chitosan de la présente invention a un effet immédiat de soulagement de la douleur, dû probablement à l'effet d'hydratation et de refroidissement, ainsi qu'à l'absence de tout caractère irritant dû à un pH acide ou basique. Cette action de soulagement de la douleur peut être renforcée et/ou prolongée de façon synergique par l'incorporation, dans l'hydrogel de la présente invention, d'un extrait de *Calendula officinalis,* bien connue pour ses vertues cicatrisantes. La Demanderesse a obtenu d'excellents résultats en utilisant un extrait hydroglycériné de *calendula officinales,* en une concentration de 0,2 à 0,5 %.

Dans la perspective de l'utilisation cosmétique de l'hydrogel et du produit de déshydratation de celui-ci, mais surtout de l'utilisation dermatologique dudit produit de déshydratation, il est souhaitable de disposer d'échantillons stériles d'hydrogel compatibles avec une utilisation pharmaceutique. La stérilisation de l'hydrogel se fait de préférence par autoclavage ou par irradiation avec un rayonnement stérilisant, par exemple une dose suffisante de rayonnement gamma.

Dans un mode de réalisation particulier de la présente invention, l'hydrogel est conditionné sous forme de doses unitaires destinées à prévenir la contamination bactérienne due à une conservation prolongée après ouverture.

La présente invention a en outre pour objet un procédé de préparation de l'hydrogel tel que défini ci-dessus. Ce procédé comprend les étapes successives suivantes :
Etape 1 - préparation d'une solution aqueuse acide d'un chitosan présentant un taux de désacétylation compris entre 85 et 95 %, ladite solution présentant un pH compris entre 4,5 et 5,5 ajusté par addition d'un acide organique de formule R-COOH, où R représente un groupe alkyle en C₁₋₄, de préférence un groupe méthyle,
Etape 2 - réaction du sel d'addition d'acide du chitosan en solution aqueuse ainsi obtenu avec 0,5 à 1 mole, par mole de motifs D-glucosamine (motifs (B)), d'un anhydride d'un diacide organique de formule HOOC-(CH₂)ₙ-COOH où n représente un nombre entier compris entre 1 et 8, de préférence entre 1 et 4 et en particulier égal à 2, puis
Etape 3 - ajustement du pH de la solution réactionnelle ainsi obtenue par addition d'une base physiologiquement acceptable jusqu'à une valeur comprise entre 6,5 et 7,2.

La concentration en chitosan de la solution de l'étape 1 est de préférence comprise entre 5 et 150 g/l, en particulier entre 10 et 50 g/l. Dans un mode de réalisation préféré, la quantité appropriée de chitosan désacetylé, sous forme de poudre, est mise en suspension dans de l'eau purifiée sous agitation, et l'on ajoute lentement l'acide organique, de préférence en une quantité équimolaire au nombre de motifs glucosamine. Cette étape, réalisée de préférence à température ambiante, peut prendre quelques dizaines de minutes. Une variante de cette étape consiste à acidifier d'abord l'eau avec la quantité appropriée d'acide et d'introduire ensuite le chitosan.

Cette étape de dissolution par acidification peut être suivie ou précédé de l'addition d'une faible quantité d'eau oxygénée, destinée, si nécessaire, à réduire la masse moléculaire du chitosan par coupure oxydante du squelette macromoléculaire. La quantité d'eau oxygénée est de préférence comprise entre 0,01 % et 0,03 %.

La réaction de l'anhydride d'acide dicarboxylique se fait de préférence à une température comprise entre 20 °C et 30 °C, sous agitation, pendant une durée comprise par exemple entre 45 minutes et 90 minutes. L'anhydride peut être ajouté en deux ou plusieurs fois et entre les différents ajouts, la solution peut être neutralisée par addition d'une base. Après addition et réaction de la totalité de l'anhydride, la solution est neutralisée progressivement avec une solution basique diluée, par exemple une solution diluée de soude ou de potasse, en prenant soin de ne pas provoquer la précipitation du chitosan par une addition trop rapide de base. Le produit obtenu ainsi est un hydrogel selon l'invention et n'a pas besoin d'être soumis à d'autres étapes de traitement ou de concentration.

La présente invention a en outre pour objet l'utilisation de l'hydrogel à base de carboxyalkylamide de chitosan décrit ci-dessus pour le traitement cosmétique de brûlures du premier degré et de brûlures non suintantes, c'est-à-dire superficielles, du deuxième degré par application dudit hydrogel sur la zone de brûlure. L'utilisation de l'hydrogel se fait par simple application topique sur les zones touchées, une ou plusieurs fois par jour, jusqu'à disparition des troubles. Il ne s'agit pas d'un traitement dermatologique car sur des brûlures du premier degré ou des brûlures non suintantes du deuxième degré, l'hydrogel agit uniquement au niveau de l'épiderme et ne vient pas en contact avec le derme sous-jacent.

La composition cosmétique contenant l'hydrogel selon la présente invention peut en outre contenir d'autres actifs cosmétiques ou des additifs, tels que notamment parfums, colorants, agents de texture.

Au cours de ses recherches visant à mettre au point l'hydrogel décrit ci-dessus, la Demanderesse a constaté que i'hydrogel pouvait être déshydraté par des procédés connus tels que la lyophilisation, l'atomisation et le séchage par pulvérisation et que le produit anhydre pouvait être utilisé tel quel, sans reconstitution préalable par addition d'eau, à des fins cosmétiques sur des brûlures du premier degré et des brûlures non suintantes du deuxième degré, et présentait en outre l'avantage de convenir très bien à une utilisation dermatologique sur des brûlures suintantes, c'est-à-dire profondes, du deuxième degré et des brûlures du troisième degré.

En effet le produit de déshydratation de l'hydrogel, à condition de présenter une granulométrie suffisamment fine, s'étale, comme l'hydrogel à partir duquel il a été préparé, facilement sur la peau et y exerce un effet de soulagement de la douleur et un effet cicatrisant. De manière assez surprenante, les produits de déshydratation de l'hydrogel selon la présente invention semblent ainsi pénétrer dans la peau sans laisser de traces visibles.

La présente invention a ainsi en outre pour objet un produit anhydre à base de carboxyalkylamide de chitosan obtenu par déshydratation de l'hydrogel décrit ci-dessus. Le procédé de déshydratation peut être n'importe quel procédé de déshydratation connu, par évaporation de l'eau de la composition sous vide et/ou sous chauffage. La Demanderesse a obtenu des produits d'excellente qualité par lyophilisation et par séchage par pulvérisation, ou séchage par atomisation, et le procédé de déshydratation est par conséquent de préférence choisi parmi ces deux derniers.

La présente invention a également pour objet ledit produit anhydre en tant que médicament et en tant que dispositif médical, de préférence pour application topique et destiné de préférence au traitement de brûlures profondes suintantes du deuxième degré et de brûlures du troisième degré.

Enfin, la présente invention a pour objet une composition cosmétique et une composition dermatologique contenant le produit anhydre décrit ci-dessus.

### Exemple 1

### Procédé de préparation d'un hydrogel de carboxyalkylamide de chitosan selon la présente invention

On introduit dans une cuve d'une capacité de 50 litres, 36 litres d'eau purifiée et on y verse lentement, sous une agitation de 2000 à 3000 tours par minute, 926.6 g de poudre de chitosan Kitomer (Marinard) ayant un degré de désacétylation de 94,4 % et un taux d'humidité de 2,87 %. Au bout d'un quart d'heure d'agitation, on ajoute 319 g d'acide acétique (environ 1 équivalent molaire par rapport au nombre de motifs glucosamine du chitosan) et on poursuit l'agitation à température ambiante pendant encore quelques minutes. On ajoute ensuite 0,1 g d'H₂O₂ à 30 % et l'on laisse reposer le mélange pendant environ 1 heure. On ajoute ensuite au mélange, sous forte agitation, environ 133 g d'anhydride succinique (environ 0,30 équivalent molaire par rapport au nombre de motifs glucosamine du chitosan) et on poursuit l'agitation pendant environ 30 minutes. La solution obtenue est neutralisée très progressivement par addition lente d'environ 160 g de soude diluée dans 1,5 litres d'eau sous agitation et sur un intervalle d'environ 15 minutes. On répète l'étape d'addition d'anhydride succinique et de neutralisation en prenant soin que le pH de la solution n'augmente pas au-delà de la valeur finale visée, comprise entre 6,5 et 7,2.

En suivant le mode opératoire ci-dessus, on obtient environ 39 litres de gel prêt à l'emploi ou prêt à être soumis à une étape de déshydratation.

### Exemple 2

### Procédé de préparation d'un gel hydroglycériné contenant de l'extrait de calendula

On mélange 1,5 litres d'extrait hydroglycériné de *Calendula officinalis* avec 3,5 litres de glycérol Ph. Eur., puis on ajoute 6 litres d'eau. La solution ainsi obtenue est mélangée avec les 39 litres d'hydrogel préparés à l'Exemple 1.

Le produit ainsi obtenu est ensuite soumis à une irradiation par des rayons gamma à 25 kGy.

### Exemple 3

### Traitement de brûlures profondes du deuxième degré avec un produit de déshydratation obtenu à partir de l'hydrogel

Une poudre stérile, obtenue par atomisation d'un hydrogel préparé conformément à l'exemple 1, constituée de particules sphériques de 10 à 30 µm, est appliquée sur trois brûlures profondes du deuxième degré, provoquées par un traitement au laser sur le dos de la main. Les brûlures ont un diamètre d'environ 5 mm. Une quatrième brûlure témoin, identique aux trois autres, ne reçoit aucun traitement.

La brûlure témoin, non traitée, met entre trois et quatre semaines pour cicatriser et disparaître.

Les trois brûlures traitées à raison de 6 applications le premier jour et de 2 applications le jour suivant ne montrent plus de signe inflammatoire au bout de 48 heures et sont invisibles au bout de quatre jours. La douleur disparaît dès la première application.

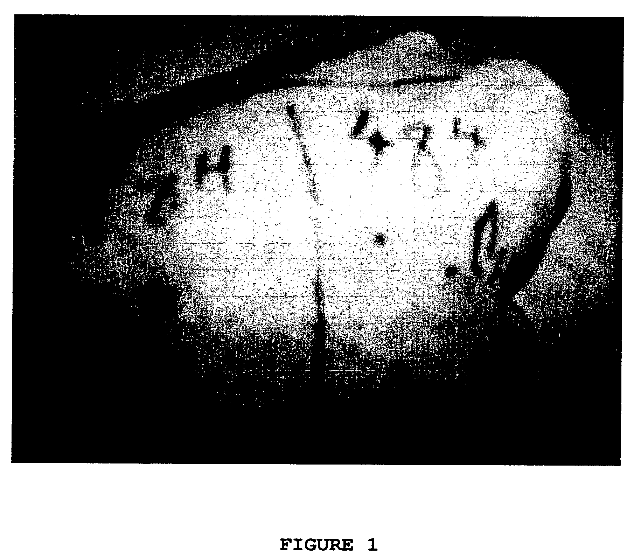

La Figure 1 montre un cliché du dos de la main au bout de 48 heures, avec la brûlure témoin à gauche et les trois brûlures traitées à droite.

### Exemple 4

### Traitement d'une brûlure du premier degré avec l'hydrogel

On traite le dos d'une main ébouillanté par le versement d'eau bouillante (brûlure du premier degré) par application de l'hydrogel de l'exemple 1 immédiatement après la brûlure (moins de 5 minutes après), puis au bout d'environ une demi-heure et de nouveau après 2 heures.

La douleur cesse dès la première application. La main reste rouge pendant environ 6 heures. Le lendemain, il n'y a plus aucune trace de brûlure.

## Revendications

1. Hydrogel de carboxyalkylamide de chitosan, **caractérisé par le fait qu'**il présente une valeur de pH proche de celui de la peau, compris entre 6,5 et 7,2, et **par le fait que** ledit carboxyalkylamide de chitosan est constitué
- de 40 à 90 % en moles, de préférence de 50 à 80 % en moles, et en particulier de 50 à 75 % en moles, rapporté au nombre total de motifs (A) et (B), de motifs N-carboxyalkylamide de D-glucosamine de formule (I) (motifs (A)) 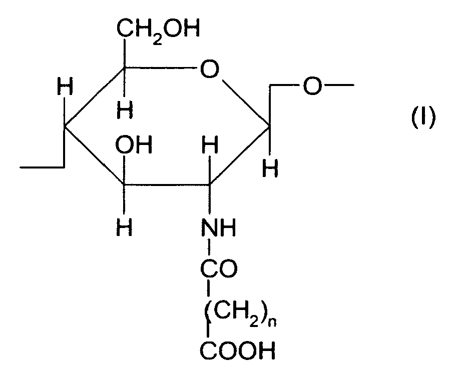 où n représente un nombre entier allant de 1 à 8, de préférence de 1 à 4, et en particulier égal à 2, ou un sel d'addition d'une base physiologiquement acceptable de celui-ci,
- de 60 à 10 % en moles, de préférence de 50 à 20 % en moles, en particulier de 50 à 25 % en moles, rapporté au nombre total de motifs (A) et (B), de motifs D-glucosamine protonés de formule (II) (motifs (B)) 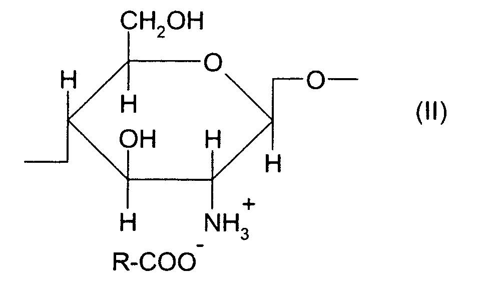 dans laquelle R représente un résidu alkyle en C₁₋₄, et
- de 5 à 15 % en moles, rapporté au nombre total de motifs (A), (B) et (C), de motifs N-acétyl-D-glucosamine (motifs (C)).

2. Hydrogel selon la revendication 1, **caractérisé par le fait que** R représente un groupe méthyle.

3. Hydrogel selon la revendication 1 ou 2, **caractérisé par le fait qu'**il présente un pH compris entre 6,8 et 7,0.

4. Hydrogel selon l'une quelconque des revendications précédentes, **caractérisé par le fait que** la concentration de carboxyalkylamide de chitosan est comprise entre 0,5 et 3 % en poids, de préférence entre 1 et 2 % en poids.

5. Hydrogel selon l'une quelconque des revendications précédentes, **caractérisé par le fait qu'**il contient en outre de 0,1 à 40 % en poids, de préférence de 0,1 à 20 % en poids, d'au moins un polyol, de préférence du glycérol.

6. Hydrogel selon l'une quelconque des revendications précédentes, **caractérisé par le fait qu'**il contient en outre un extrait hydroglycériné de *calendula officinales.*

7. Hydrogel selon l'une quelconque des revendications précédentes, **caractérisé par le fait qu'**il est stérile.

8. Hydrogel selon l'une quelconque des revendications précédentes, **caractérisé par le fait qu'**il est conditionné sous forme d'une dose unitaire.

9. Procédé de préparation d'un hydrogel selon l'une quelconque des revendications précédentes, **caractérisé par le fait qu'**il comprend les étapes successives suivantes
- préparation d'une solution aqueuse acide d'un chitosan présentant un taux de désacétylation compris entre 85 et 95 %, ladite solution présentant un pH compris entre 4,5 et 5,5 obtenu par addition d'un acide organique de formule R-COOH, où R représente un groupe alkyle en C₁₋₄, de préférence un groupe méthyle,
- réaction du sel d'addition d'acide du chitosan en solution aqueuse ainsi obtenu avec 0,5 à 1 mole, par mole de motifs D-glucosamine (motifs (B)), d'un anhydride d'un diacide organique de formule HOOC-(CH₂)ₙ-COOH où n représente un nombre entier compris entre 1 et 8, de préférence entre 1 et 4 et en particulier égal à 2, puis
- ajustement du pH de la solution réactionnelle ainsi obtenue par addition d'une base physiologiquement acceptable jusqu'à une valeur comprise entre 6,5 et 7,2.

10. Utilisation d'un hydrogel selon l'une quelconque des revendications 1 à 8 pour le traitement cosmétique de brûlures du premier degré et de brûlures superficielles non suintantes de deuxième degré par application dudit hydrogel sur la zone de brûlure.

11. Produit anhydre à base de carboxyalkylamide de chitosan obtenu par déshydratation de l'hydrogel selon l'une quelconque des revendications 1 à 8, le procédé de déshydratation étant choisi de préférence parmi la lyophilisation et le séchage par pulvérisation.

12. Produit anhydre selon la revendication 11, en tant que médicament, de préférence en tant que médicament destiné au traitement de brûlures profondes suintantes du deuxième degré ou de brûlures du troisième degré.

13. Produit anhydre selon la revendication 11, en tant que dispositif médical, de préférence destiné au traitement de brûlures profondes suintantes du deuxième degré ou de brûlures du troisième degré.

14. Composition dermatologique contenant un produit anhydre selon la revendication 11.

15. Composition cosmétique contenant un produit anhydre selon la revendication 11.

## Claims

1. Hydrogel of chitosan carboxyalkylamide, **characterised in that** it has a pH value close to that of the skin, comprised between 6.5 and 7.2, and **in that** the said chitosan carboxyalkylamide is constituted by
- from 40 to 90 mole %, preferably from 50 to 80 mole %, and in particular from 50 to 75 mole %, relative to the total number of (A) and (B) units, of D-glucosamine N-carboxyalkylamide units of formula (I) ((A) units) 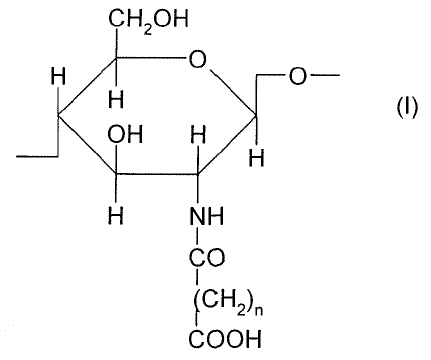 where n represents an integer ranging from 1 to 8, preferably from 1 to 4, and in particular equal to 2, or a physiologically acceptable base addition salt thereof,
- from 60 to 10 mole %, preferably from 50 to 20 mole %, in particular from 50 to 25 mole %, relative to the total number of (A) and (B) units, of protonated D-glucosamine units of formula (II) ((B) units) 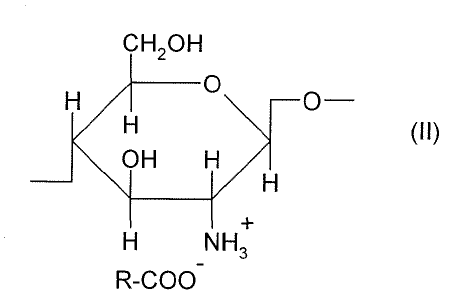 wherein R represents a C₁₋₄ alkyl residue, and
- from 5 to 15 mole %, relative to the total number of (A), (B) and (C) units, of N-acetyl-D-glucosamine units ((C) units).

2. Hydrogel according to claim 1, **characterised in that** R represents a methyl group.

3. Hydrogel according to claim 1 or 2, **characterised in that** it has a pH comprised between 6.8 and 7.0.

4. Hydrogel according to any one of the previous claims, **characterised in that** the concentration of chitosan carboxyalkylamide is comprised between 0.5 and 3% by weight, preferably between 1 and 2% by weight.

5. Hydrogel according to any one of the previous claims, **characterised in that** it further contains from 0.1 to 40% by weight, preferably from 0.1 to 20% by weight, of at least one polyol, preferably of glycerol.

6. Hydrogel according to any one of the previous claims, **characterised in that** it further contains a water-glycerine extract of *calendula officinalis.*

7. Hydrogel according to any one of the previous claims, **characterised in that** it is sterile.

8. Hydrogel according to any one of the previous claims, **characterised in that** it is packed in the form of a unit dose.

9. Process for the preparation of a hydrogel according to any one of the previous claims, **characterised in that** it comprises the following consecutive stages
- preparation of an acidic aqueous solution of a chitosan having a deacetylation level comprised between 85 and 95%, the said solution having a pH comprised between 4.5 and 5.5 obtained by the addition of an organic acid of formula R-COOH, where R represents a C₁₋₄ alkyl group, preferably a methyl group,
- reaction of the acid addition salt of chitosan in aqueous solution thus obtained with 0.5 to 1 mole, per mole of D-glucosamine units ((B) units), of an anhydride of an organic diacid of formula HOOC-(CH₂)ₙ-COOH where n represents an integer comprised between 1 and 8, preferably between 1 and 4 and in particular equal to 2, then
- adjustment of the pH of the reaction solution thus obtained to a value comprised between 6.5 and 7.2 by the addition of a physiologically acceptable base.

10. Utilisation of a hydrogel according to any one of claims 1 to 8 for the cosmetic treatment of first degree burns and of non-weeping, superficial second degree burns by the application of the said hydrogel to the burn area.

11. Anhydrous product based on chitosan carboxyalkylamide obtained by the dehydration of the hydrogel according to any one of claims 1 to 8, the dehydration process preferably being selected from lyophilisation and spray drying.

12. Anhydrous product according to claim 11, as a medicament, preferably as a medicament intended for the treatment of deep, weeping second degree burns or of third degree burns.

13. Anhydrous product according to claim 11, as a medical device, preferably intended for the treatment of deep, weeping second degree burns or of third degree burns.

14. Dermatological composition containing an anhydrous product according to claim 11.

15. Cosmetic composition containing an anhydrous product according to claim 11.

## Patentansprüche

1. Chitosan-Carboxyalkylamid-Hydrogel, **dadurch gekennzeichnet, dass** es einen pH-Wert nahe dem der Haut, zwischen 6,5 und 7,2, aufweist, und **dadurch**, dass das Chitosan-Carboxyalkylamid zusammengesetzt ist
- aus von 40 bis 90 Mol-%, bevorzugt von 50 bis 80 Mol-%, und insbesondere von 50 bis 75 Mol-%, bezogen auf die Gesamtanzahl an Motiven (A) und (B), an N-Carboxyalkylamid-D-Glucosaminmotiven der Formel (I) (Motive (A)) 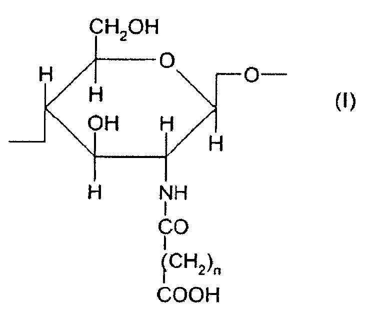 worin n eine ganze Zahl von 1 bis 8, bevorzugt von 1 bis 4, und insbesondere gleich 2, darstellt oder ein physiologisch akzeptables Basenadditionssalz davon,
- von 60 bis 10 Mol-%, bevorzugt von 50 bis 20 Mol-%, insbesondere von 50 bis 25 Mol-%, bezogen auf die Gesamtanzahl an Motiven (A) und (B), an protonierten D-Glucosaminmotiven der Formel (II) (Motive (B)) 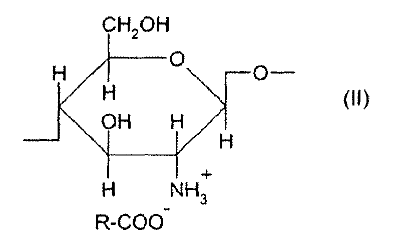 worin R einen C₁₄-Alkylrest darstellt und
- von 5 bis 15 Mol-%, bezogen auf die Gesamtanzahl an Motiven (A), (B) und (C), an N-Acetyl-D-glucosaminmotiven (Motive (C)).

2. Hydrogel gemäß Anspruch 1, **dadurch gekennzeichnet, dass** R eine Methylgruppe darstellt.

3. Hydrogel gemäß Anspruch 1 oder 2, **dadurch gekennzeichnet, dass** es einen pH-Wert zwischen 6,8 und 7,0 aufweist.

4. Hydrogel gemäß einem der vorhergehenden Ansprüche, **dadurch gekennzeichnet, dass** die Konzentration von Chitosan-Carboxyalkylamid zwischen 0,5 und 3 Gewichts-%, bevorzugt zwischen 1 und 2 Gewichts-% ist.

5. Hydrogel gemäß einem der vorhergehenden Ansprüche, **dadurch gekennzeichnet, dass** es außerdem von 0,1 bis 40 Gewichts-%, bevorzugt von 0,1 bis 20 Gewichts-%, mindestens eines Polyols, bevorzugt von Glycerin, enthält.

6. Hydrogel gemäß einem der vorhergehenden Ansprüche, **dadurch gekennzeichnet, dass** es außerdem einen Hydroglycerinextrakt von *Calendula officinalis* enthält.

7. Hydrogel gemäß einem der vorhergehenden Ansprüche, **dadurch gekennzeichnet, dass** es steril ist.

8. Hydrogel gemäß einem der vorhergehenden Ansprüche, **dadurch gekennzeichnet, dass** es in Form einer Dosiseinheit abgepackt ist.

9. Verfahren zur Herstellung eines Hydrogels gemäß einem der vorhergehenden Ansprüche, **dadurch gekennzeichnet, dass** es die folgenden aufeinanderfolgenden Schritte umfasst:
- Herstellung einer sauren wässrigen Lösung eines Chitosans, das einen Deacetylierungsgrad zwischen 85 und 95% aufweist, wobei die Lösung einen pH-Wert zwischen 4,5 und 5,5 aufweist, erhalten durch Zugabe einer organischen Säure der Formel R-COOH, worin R eine C₁₋₄-Alkylppe, bevorzugt eine Methylgruppe darstellt,
- Umsetzen des so erhaltenen Säureadditionssalzes des Chitosans in wässriger Lösung mit 0,5 bis 1 Mol, pro Mol an D-Glucosaminmotiven (Motive (B)), eines Anhydrids einer organischen Disäure der Formel HOOC-(CH₂)ₙ-COOH, worin n eine ganze Zahl von 1 bis 8, bevorzugt von 1 bis 4, und insbesondere gleich 2, darstellt, dann
- Einstellen des pH-Werts der so erhaltenen Reaktionslösung durch Zugabe einer physiologisch akzeptablen Base bis zu einem Wert zwischen 6,5 und 7,2.

10. Verwendung eines Hydrogels gemäß einem der Ansprüche 1 bis 8 zur kosmetischen Behandlung von Verbrennungen ersten Grades und von oberflächlichen nicht nässenden Verbrennungen zweiten Grades durch Auftragen des Hydrogels auf das Verbrennungsgebiet.

11. Wasserfreies Produkt auf der Basis von Chitosan-Carboxyalkylamid, erhalten durch Dehydration des Hydrogels gemäß einem der Ansprüche 1 bis 8, wobei das Dehydrationsverfahren bevorzugt aus der Lyophilisierung und der Trocknung durch Pulverisieren ausgewählt ist.

12. Wasserfreies Produkt gemäß Anspruch 11, als Medikament, bevorzugt als Medikament, das für die Behandlung von tiefen nässenden Verbrennungen zweiten Grades oder von Verbrennungen dritten Grades bestimmt ist.

13. Wasserfreies Produkt gemäß Anspruch 11, als medizinische Vorrichtung, die bevorzugt für die Behandlung von tiefen nässenden Verbrennungen zweiten Grades oder von Verbrennungen dritten Grades bestimmt ist.

14. Dermatologische Zusammensetzung, enthaltend ein wasserfreies Produkt gemäß Anspruch 11.

15. Kosmetische Zusammensetzung, enthaltend ein wasserfreies Produkt gemäß Anspruch 11.
